(19) Europäisches Patentamt
European Patent Office
Office européen des brevets

(11) Publication number: **0 330 467 A1**

(12) # EUROPEAN PATENT APPLICATION

(21) Application number: 89301771.5

(22) Date of filing: 23.02.89

(51) Int. Cl.[4]: **C 07 D 401/06**
C 07 D 413/06,
C 07 D 211/26,
C 07 D 217/14,
A 61 K 31/445, A 61 K 31/47

(30) Priority: 23.02.88 GB 8804105
23.02.88 GB 8804108

(43) Date of publication of application:
30.08.89 Bulletin 89/35

(84) Designated Contracting States:
AT BE CH DE ES FR GB GR IT LI LU NL SE

(71) Applicant: GLAXO GROUP LIMITED
Clarges House 6-12 Clarges Street
London W1Y 8DH (GB)

(72) Inventor: Hays, Norman Frank
201 Whitehill Road
Hitchin Hertfordshire (GB)

Scopes, David Ian Carter
Gable View The Causeway Furneux
Pelham Hertfordshire (GB)

Brown, Dearg Sunderland
26 Chapmore End
Ware Hertfordshire (GB)

Bays, David Edmund
9 Windmill Field
Ware Hertfordshire (GB)

Hays, Ann Gail
5 Sandringham Road
Potters Bar Hertfordshire (GB)

(74) Representative: Pett, Christopher Phineas et al
Frank B.Dehn & Co. Imperial House 15-19 Kingsway
London WC2B 6UZ (GB)

Claims for the following Contracting States: ES + GR.
A request for correction of a clerical error has been filed pursuant to Rule 88 EPC. A decision on the request will be taken during the proceedings before the Examining Division (Guidelines for Examination in the EPO, A-V, 2.2).

(54) **Heterocyclic compounds.**

(57) Compounds are disclosed of formula (I)

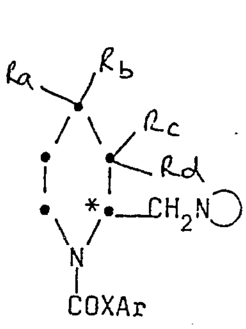

(I)

wherein
$R_a$, $R_b$, $R_c$ and $R_d$ represent hydrogen atoms or $R_a$ and $R_c$ together form a bond and $R_b$ and $R_d$ together form -CH=CH-CH=CH-; 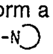 represents a 5-membered (optionally containing an oxygen atom adjacent to the nitrogen) or a 6-membered ring, which ring optionally contains one unit of unsaturation and which is unsubstituted or substituted by hydroxy, hydroxymethyl, oxo, optionally substituted methylidene, $-COR_1$ (where $R_1$ is $C_{1-6}$ alkyl, $-OR_2$ or $-NHR_2$, and $R_2$ is hydrogen, $C_{1-6}$ alkyl, aryl or ar($C_{1-6}$)alkyl) or $=NOR_3$ (where $R_3$ is $C_{1-6}$ alkyl);
X represents a direct bond, $-CH_2-$ or $-CH_2O-$;
Ar represents a substituted phenyl moiety (provided that when -N does not contain an oxygen atom and is unsubstituted, Ar contains more than one unsaturated ring);
and physiologically acceptable salts and solvates thereof.

The compounds are indicated as useful for the treatment of pain and cerebral ischaemia.

Processes and intermediates for their preparation and pharmaceutical compositions containing them are also disclosed.

EP 0 330 467 A1

**Description**

## HETEROCYCLIC COMPOUNDS

This invention relates to heterocyclic derivatives, to processes for their preparation, to pharmaceutical compositions containing them and to their medical use. In particular, the invention relates to compounds which act as agonists at kappa opioid receptors.

Compounds which are kappa opioid receptor agonists have been indicated in the art for the treatment of a number of conditions and have been described, for example, as analgesics, as diuretics and in the treatment of cerebral ischaemia. Opioid analgesia is generally thought to be mediated by either mu or kappa receptors in the brain (see, for example, Tyers M.B., Br. J. Pharmacol, (1980), 69, 503-512). Most existing clinically used opioid analgesics such as morphine and codeine act as mu-receptor agonists. However, these compounds have undesirable and potentially dangerous dependence forming side effects. There is thus a need for a strong analgesic with low dependence liability and a compound which is a selective kappa-receptor agonist would fulfil such a role.

Cerebral ischaemia or lack of blood flow in the brain, may result from a number of conditions, including, for example, stroke, head injuries or brain tumour. The resulting lack of oxygen to the brain cells causes neuronal damage and depending on the region of the brain involved, death or permanent disability may occur.

We have now found a novel group of heterocyclic derivatives which are selective kappa opioid receptor agonists. These compounds are therefore of interest in the treatment of conditions where the underlying aetiology indicates that treatment with a kappa opioid receptor agonist would be beneficial.

Thus, the present invention provides a compound of formula (I):

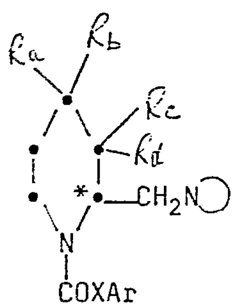

(I)

wherein

$R_a$, $R_b$, $R_c$ and $R_d$ represent hydrogen atoms or $R_a$ and $R_c$ together form a bond and $R_b$ and $R_d$ together form -CH=CH-CH=CH-; 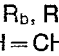 represents a 5-membered (optionally containing an oxygen atom adjacent to the nitrogen) or a 6-membered ring, which ring optionally contains one unit of unsaturation and which is unsubstituted or substituted by hydroxy, hydroxymethyl, oxo, optionally substituted methylidene, $-COR_1$ (where $R_1$ is $C_{1-6}$ alkyl, $-OR_2$ or $-NHR_2$, and $R_2$ is hydrogen, $C_{1-6}$ alkyl, aryl or ar($C_{1-6}$)alkyl) or $=NOR_3$ (where $R_3$ is $C_{1-6}$ alkyl);

X represents a direct bond, $-CH_2-$ or $-CH_2O-$;

Ar represents a substituted phenyl moiety (provided that when -N does not contain an oxygen atom and is unsubstituted, Ar contains more than one unsaturated ring);

and physiologically acceptable salts and solvates thereof.

As used herein, a $C_{1-6}$ alkyl group or the alkyl moiety of an ar($C_{1-6}$)alkyl group may be straight or branched chain and is conveniently $C_{1-4}$ alkyl for example methyl or ethyl. An aryl group or the aryl moiety of an ar($C_{1-6}$)alkyl group is preferably phenyl. It will be appreciated that where 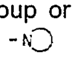 contains one unit of unsaturation this will not be attached to a carbon atom adjacent to the nitrogen atom.

The term 'optionally substituted methylidene' is intended to cover methylidene substituted by any substituent conventional in the art. In the compounds of formula (I), the methylidene group may conveniently be substituted to form a conjugated system. Suitable substituents which form a conjugated system with the methylidene double bond include, for example, nitrile, phenyl, carboxyl and amido Alternatively, the methylidene group may conveniently be substituted, for example, by $C_{1-6}$ alkyl, ar($C_{1-6}$) alkyl, a $C_{1-6}$ hydroxy alkyl group such as hydroxymethyl, a $C_{1-6}$ carboxyalkyl group such as methoxycarbonylethyl or a $C_{1-6}$ amidoalkyl group such as aminocarbonylethyl.

The term 'a substituted phenyl moiety' is intended to cover a phenyl moiety substituted by one or more conventional substituents in the art, which substituents may together form a second ring containing one or more units of unsaturation. In the compounds of formula (I), Ar conveniently represents a phenyl moiety which is substituted by one or more $C_{1-6}$ alkyl groups or electron-withdrawing substituents. Suitable electron-withdrawing substituents include, for example, halogen (for example, fluorine, chlorine or bromine), $-CF_3$ or $-NO_2$. Ar is preferably phenyl substituted at the meta and/or para positions by one or more halogens,

for example chlorine and is typically a 3,4-dichlorophenyl moiety.

Where two substituents on the phenyl ring form a second ring, Ar may suitably represent naphthyl, for example 1-naphthyl or 2-naphthyl.

In one preferred class of compounds of formula (I), $R_a$, $R_b$, $R_c$ and $R_d$ represent hydrogen atoms.

In another preferred class of compounds of formula (I), $R_a$ and $R_c$ form a bond and $R_b$ and $R_c$ together form -CH=CH-CH=CH-.

-N◯ may suitably represent a pyrrolidine, tetrahydropyridine or isoxazolidine ring which may be optionally substituted, for example, by $-CO_2CH_3$, $-CH=CH_2$, phenylpropylidene, $-CH_2OH$, $=CH_2$, preferably by $=NOCH_3$,$-CONH_2$ or $=CHCONH_2$, or more preferably by -OH = O, =CHCN or $=CHCO_2CH_3$. Where -N◯ is substituted, the substituent is preferably attached to the carbon atom β to the nitrogen atom. Where -N◯ is substituted by a substituted methylidene group the substituent on the methylidene is preferably in the Z configuration with respect to the ring nitrogen.

In a further preferred class of compounds of formula (I), -N◯ represents a pyrrolidine ring which is unsubstituted or substituted by -OH, =O, =CHCN or $=CHCO_2CH_3$.

In an alternative preferred class of compounds of formula (I) -N◯ represents a pyrrolidine ring which is substituted by =O, =CHCN or $=CHCO_2CH_3$ when $R_a$, $R_b$, $R_c$ and $R_d$ represent hydrogen atoms.

In another preferred class of compounds of formula (I) when $R_a$ and $R_c$ form a bond and $R_b$ and $R_d$ together form -CH=CH-CH=CH-, -N◯ represents pyrrolidine ring which is substituted by -OH.

X preferably represents $-CH_2-$

In a further preferred class of compounds of formula (I), Ar represents a halosubstituted phenyl moiety, in particular a chlorosubstituted phenyl moiety such as 3,4-dichlorophenyl.

In another preferred class of compounds of formula (I), Ar represents a naphthyl moiety.

A further preferred group of compounds falling within the scope of formula (I) is that in which $R_a$, $R_b$, $R_c$ and $R_d$ represent hydrogen atoms; -N◯ represents a pyrrolidine ring which is substituted by =O, =CHCN or $=CHCO_2CH_3$; X represents $-CH_2-$; and Ar represents halosubstituted phenyl; and physiologically acceptable salts or solvates thereof. Particularly preferred compounds falling within this group are those in which Ar represents chlorosubstituted phenyl.

Another preferred group of compounds falling within the scope of formula (I) is that in which $R_a$ and $R_c$ form a bond and $R_b$ and $R_d$ together form -CH=CH-CH=CH-; -N◯ represents a pyrrolidine ring which is substituted by -OH; X represents $-CH_2$; Ar represents halosubstituted phenyl; and physiologically acceptable salts or solvates thereof. Particularly preferred compounds falling within this group are those in which Ar represents chlorosubstituted phenyl.

Preferred compounds according to the invention include:

1-[(3,4-Dichlorophenyl)acetyl]-2-[(3-hydroxy-1-pyrrolidinyl)methyl]-piperidine;
1-[(3,4-Dichlorophenyl)acetyl]-2-[(3-methylene-1-pyrrolidinyl)-methyl]piperidine;
1-[(3,4-Dichlorophenyl)acetyl]-2-[[3-(methoxyimino)-1-pyrrolidinyl]-methyl]piperidine;
Methyl [1-[[1-[(3,4-dichlorophenyl)acetyl]-2-piperidinyl]methyl]-3-pyrrolidinylidene]acetate;
1-[[1-[(3,4-Dichlorophenyl)acetyl]-2-piperidinyl]methyl]-1,2,5,6-tetrahydro-3-pyridinecarboxamide;
Methyl 1-[[1-[(3,4-dichlorophenyl)acetyl]-2-piperidinyl]methyl]-1,2,5,6-tetrahydro-3-pyridinecarboxylate;
1-(1-Naphthalenylacetyl)-2-(1-pyrrolidinylmethyl)piperidine;
and physiologically acceptable salts and solvates thereof.

Particularly preferred compounds according to the invention include:

1-[(3,4-Dichlorophenyl)acetyl]-2-[(3-oxo-1-pyrrolidinyl)methyl]-piperidine;
2-[[3-(Cyanomethylene-1-pyrrolidinyl)methyl]-1-[3,4-dichlorophenyl)-acetyl]piperidine;
5-[(3,4-Dichlorophenyl)acetyl]-1,2,3,4-tetarhydro-1-[3-(hydroxy-1-pyrrolidinyl)methyl]isoquinoline;
and physiologically acceptable salts and solvates thereof.

Compounds of formula (I) contain at least one chiral centre (shown in formula (I) by *) and may exist in more than one stereoisomeric form. The invention includes within its scope all enantiomers, diastereomers and mixtures thereof. The preferred stereoisomeric form is that represented by formula Ia :

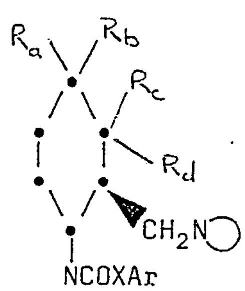

(Ia)

Wherein $R_a$, $R_b$, $R_c$, $R_d$, N◯ , X and Ar are as defined herein for formula (I).

The invention also embraces all geometric isomers of compounds of formula (I).

Suitable physiologically acceptable salts are those conventionally known in the art. Examples of physiologically acceptable salts include acid addition salts formed with inorganic acids, such as hydrochlorides, hydrobromides, phosphates and sulphates, and with organic acids, for example tartrates, maleates, fumarates, succinates and sulphonates. Other salts which are not pharmaceutically acceptable, may be useful in the preparation of compounds of formula (I) and these form a further part of the invention.

Compounds falling within formula (I) have been shown to have analgesic activity using standard laboratory animal tests such as the mouse acetylcholine writhing test (M. B. Tyers, Brit. J. Pharacol, 1980, 69, 503-512) or the rat paw pressure test. Furthermore, their selective kappa receptor activity has been demonstrated in vitro in the field stimulated rabbit vas deferens preparation using the procedure described by A. G. Hayes and A. Kelly, Eur. J. Pharmacol, 110, 317-322 (1985). Compounds of the invention and their physiologically acceptable salts and solvates thus possess analgesic activity with the potential for low dependence liability and are therefore useful in the relief of pain.

Compounds of the invention are also of value in protecting against neuronal damage resulting from cerebral ischaemia which may be demonstrated for example, using the standard laboratory bilateral carotid occlusion models. Thus, compounds of the invention and their physiologically acceptable salts and solvates are also useful in treating or relieving the effects of cerebral ischaemia.

Accordingly, the invention also provides a compound of formula (I) or a physiologically acceptable salt or solvate thereof for use in medicine, in particular for the treatment of conditions where kappa agonists are indicated, (for example as analgesics and in the treatment of cerebral ischaemia).

In an alternative or further aspect there is provided a method of treatment of a mammal, including man, comprising administration of an effective amount of a compound of formula (I) or a physiologically acceptable salt thereof in particular in the treatment of conditions where the use of akappa receptor agonist is indicated.

It will be appreciated that the compounds of the invention will primarily be of use in the alleviation of established symptoms but prophylaxis is not excluded.

Compounds of formula (I) may be administered as the raw chemical but the active ingredient is preferably presented as a pharmaceutical formulation. The active ingredient may conveniently be presented in unit dose form.

According to another aspect, the invention provides a pharmaceutical composition comprising at least one compound of formula (I) or a physiologically acceptable salt or solvate thereof and formulated for administration by any convenient route conventional in the art. Such compositions are preferably in a form adapted for use in medicine, in particular human medicine, and may conveniently be formulated in conventional manner using one or more pharmaceutically acceptable carriers or excipients. Compounds according to the invention may conveniently be formulated for oral or parenteral administration.

For oral administration, the pharmaceutical compositions may take the form of, for example, tablets or capsules prepared by conventional means with pharmaceutically acceptable excipients such as binding agents (for example pregelatinised maize starch, polyvinylpyrrolidone or hydroxypropyl methylcellulose); fillers (for example lactose, microcrystalline cellulose or calcium phosphate); lubricants (for example magnesium stearate, talc or silica); disintegrants (for example potato starch or sodium starch glycollate); or wetting agents (for example sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (for example sorbitol syrup, methyl cellulose or hydrogenated edible fats); emulsifying agents (for example lecithin or acacia); non-aqueous vehicles (for example almond oil, oily esters or ethyl alcohol); and preservatives (for example methyl or propyl-p-hydroxybenzoates or sorbic acid).

The compounds of the invention may be formulated for parenteral administration by injection preferably intranevous or subcutaneous injection, for example by bolus injection or continuous intravenous infusions. Where the compounds are administered by continuous intravenous infusion this is conveniently sequential to a bolus injection. Formulations for injection may be presented in unit dosage form, for example, in ampoules or in multi-dose containers, with an added preservative.

The compositions may take such forms as suspensions, solutions or emulsions in oily or aqueous vehicles, and may contain formulatory agents such as suspending, stabilising and/or dispersing agents. Alternatively, the active ingredient may be in powder form for constitution with a suitable vehicle, for example sterile pyrogen-free water, before use.

It will be appreciated that the precise dose administered will depend on the age and condition of the patient, the particular compound used, and the frequency and route of administration. The compounds may be administered in single or divided doses and may be administered one or more times, for example 1 to 4 times per day.

A proposed dose of the compounds of the invention for the relief of pain or the treatment of cerebral ischaemia is 0.01 to 100 mg/kg body weight, preferably 0.01 to 10mg/kg body weight, most preferably 0.1 to 10 mg/kg body weight per day.

The invention also provides the use of a compound of formula (I) or a physiologically acceptable salt or solvate thereof in the manufacture of a medicament for the treatment of conditions where kappa receptor agonists are indicated such as pain and cerebral ischaemia.

According to another aspect of the invention, compounds of formula (I) and physiologically acceptable salts and solvates, thereof may be prepared by the general methods outlined below. In the following methods, $R_a$, $R_b$, $R_c$, $R_d$, 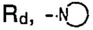 ,X and Ar are as defined for formula (I) unless otherwise indicated.

It will be appreciated that in the methods for preparing compounds of formula (I) given below, it may be necessary or desirable to protect one or more sensitive groups in the molecule to prevent undesirable side reactions. Thus, a reaction step involving deprotection of a protected derivative of a compound of the invention may be required subsequent to any of the processes described below. Protection and deprotection may be effected using conventional procedures as described, for example, in 'Protective Groups in Organic Synthesis', T. W. Greene (John Wiley & Sons, 1981).

According to one general process (A), compounds of formula (I) may be prepared by reacting a compound of formula (II)

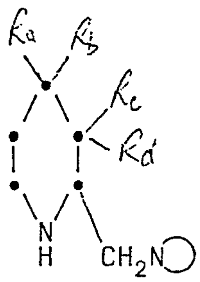

(II)

with a reagent serving to introduce the group -COXAr.

Thus, for example, compounds of formula (I) may be prepared by reacting a compound of formula (II) with an acid $ArXCO_2H$ or an acylating agent corresponding thereto or a salt thereof.

Suitable acylating agents corresponding to the acid $ArXCO_2H$ which may conveniently be used include, for example, acid halides (for example acid chlorides), alkyl esters (for example, methyl or ethyl esters) and mixed anhydrides. Such acylating agents may conveniently be prepared from the acid itself by conventional methods.

The reaction of a compound of formula (II) with an acid $ArXCO_2H$ is desirably effected in the presence of a coupling agent such as carbonyl diimidazole, dicyclohexylcarbodiimide or diphenylphosphoryl azide in a suitable reaction medium and conveniently at a temperature of from -50 to +50°C, preferably at room temperature. The reaction may be effected in a suitable reaction medium such as an ether (for example tetrahydrofuran), a haloalkane (for example, dichloromethane), a nitrile (for example acetonitrile), an amide (for example dimethylformamide) or mixtures thereof.

The reaction of a compound of formula (II) with an acylating agent corresponding to the acid $ArXCO_2H$ may conveniently be effected using the reaction conditions described above and optionally in the presence of a base. Suitable bases which may be employed include, for example, organic bases such as pyridine or triethylamine or inorganic bases such as calcium carbonate or sodium bicarbonate.

Compounds of formula (II) wherein $R_a$, $R_b$, $R_c$ and $R_d$ represent hydrogen atoms may themselves be prepared, for example, from compounds of formula (III)

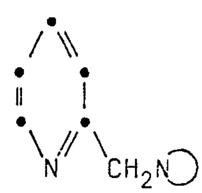

(III)

by reduction using standard conditions.

The reduction may conveniently be carried out in the presence of hydrogen and a metal catalyst such as palladium, Raney nickel, platinum, platinum oxide or rhodium which may be supported, for example, on charcoal.

Compounds of formula (III) are either known compounds or may be prepared from known compounds by conventional methods.

Alternatively, compounds of formula (II) may be prepared from compounds of formula (IV)

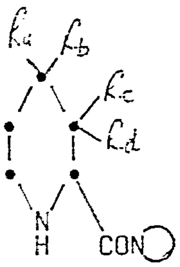

(IV)

by reduction using a suitable reducing agent, for example, a metal hydride such as lithium aluminium hydride in a solvent such as tetrahydrofuran.

Compounds of formula (IV) are either known or may be prepared from known compounds by conventional methods.

According to another general process (B), compounds of formula (I) may be prepared by reductive amination of a compound of formula (V)

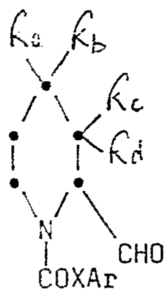

(V)

with an amine -HN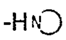 in the presence of a suitable reducing agent.

The reduction may be effected using an alkali metal or alkaline earth metal borohydride or cyanoborohydride (for example sodium borohydride or cyanoborohydride) in a suitable solvent, for example an alcohol such as methanol and at a suitable temperature, conveniently room temperature. The reaction may optionally be performed in the presence of an acid such as acetic acid.

Alternatively, the reduction may be effected catalytically, for example, using hydrogen in the presence of a metal catalyst such as Raney nickel, platinum, platinum oxide, palladium or rhodium which may by supported, for example, on charcoal. The reaction may conveniently be carried out in a suitable solvent such as an alcohol (for example ethanol), an amide (for example dimethylformamide) an ether (for example tetrahydrofuran) at a suitable temperature and optionally in the presence of an acid catalyst.

Compounds of formula (V) may be prepared, for example, from compounds of formula (VI)

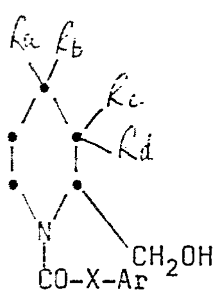

(VI)

by oxidation using conventional methods, for example using an oxidising agent such as chromium trioxide in a suitable solvent, for example pyridine.

Compounds of formula (VI) may themselves be prepared from the corresponding compound of formula (VII)

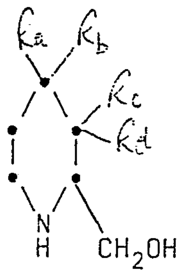

(VII)

by methods analogous to those described for general process (A) above.

According to a further general process (C), a compound of formula (I) according to the invention may be converted into another compound of the invention using conventional procedures.

According to one embodiment of process (C), a compound of formula (I) containing an oxo group may be converted into the corresponding optionally substituted methylidene derivative by reaction with an appropriate Wittig reagent, for example a phosphonate (for example trimethylphosphonoacetate) or a phosphorane prepared by reacting an appropriate triarylphosphonium salt (such as methyltriphenylphosphonium bromide) with a base. Suitable bases which may be used include, for example, alkali metal hydrides such as sodium hydride, alkali metal alkoxides such as sodium or potassium t-butoxide or alkali lithiums such as n-butyl lithium. The reaction may conveniently be carried out in a solvent such as an ether, for example tetahydrofuran, and at a temperature of from -70° to +50°.

Compounds of formula (I) containing a hydroxy group can conveniently be prepared by reduction of the corresponding oxo compound using a suitable reducing agent such as an alkali metal borohydride (for example sodium borohydride) or a metal hydride (for example diisobutyl aluminium hydride or lithium aluminium hydride) in a suitable solvent (for example, an alcohol such as ethanol or a hydrocarbon solvent such as toluene).

Compounds of formula (I) containing an oxo group may be prepared by oxidation of the corresponding alcohol using a suitable oxidising agent, for example an acid anhydride or acid chloride complex with dimethylsulphoxide (such as oxalylchloride- dimethylsulphoxide) in a solvent such as dichloromethane, conveniently at low temperature followed by treatment with a base such as triethylamine.

Compounds of formula (I) containing an oxime substituent may conveniently be prepared from the corresponding oxo derivative by conventional oximation procedures, for example by reaction with an appropriate amine $R_3ONH_2$ in a suitable solvent such as pyridine, conveniently at room temperature.

An amido substituent may be prepared from the corresponding carboxy substituted compound of formula (I) according to the method of general process (A) above.

As well as being employed as the last main step in the reaction sequence, the general methods discussed above may also be used to introduce a desired group at any intermediate stage in the preparation of compounds of formula (I). Thus, for example, the required group at the 2-position of the piperidine nucleus in the compounds of the invention can be introduced before or after acylation to introduce the -COXAr moiety. It will be appreciated that the sequence of reactions will be chosen such that the reaction conditions do not affect groups present in the molecule which are required in the final product.

The general processes described above may yield the product of the general formula (I) as an individual stereoisomer or as a mixture of stereoisomers. Diastereoisomers may be separated at any convenient point in the overall synthesis by conventional methods e.g. chromatography. Specific enantiomers may be obtained by resolution of a racemic mixture at any convenient point in the overall synthesis by the use of conventional methods, see for example "Stereochemistry of Carbon Compounds" by E.L. Eliel (McGraw Hill, 1962).

Where it is desired to isolate a compound of the invention as a salt, thus may be formed by conventional methods, for example by treatment with an acid or base in a suitable solvent such as an ether (for example diethyl ether), a nitrile (for example acetonitrile), a ketone (for example acetone) a halogenated hydrocarbon (for example dichloromethane) or an ester (for example ethyl acetate). Salts may also be formed by conversion of one salt into another using conventional methods.

Solvates of compounds of formula (I) may conveniently be prepared by crystallisation or recrystallisation from an appropriate solvent.

Thus the product of any of processes (A) to (C) above may be subjected to one or two further reactions comprising

(i) converting a compound of formula (I) or a salt thereof into a physiologically acceptable salt or solvate thereof

(ii) resolution of a racemic mixture to give a specific enantiomer.

The invention is further illustrated by the following non-limiting examples.

All temperatures are in °C. Chromatography was carried out in the conventional manner using silica gel (Merck, 7729) or by flash column chromatography on silica (Merck 9385) and thin layer chromatography (t.l.c.) on silica except where otherwisestated. Dried refers to drying with $Na_2SO_4$ unless otherwise indicated. IPA is

iodoplatinic acid.

Intermediate 1

1-[(3,4-Dichlorophenyl)acetyl]-2-piperidinemethanol

A solution of 2-piperidinemethanol (0.56g) in acetonitrile (10ml) was added to a solution of 3,4-dichlorophenylacetic acid (1.02g) and 1,1′- carbonyldiimidazole (0.81g) in acetonitrile (20ml) and the mixture stirred at 22°, under nitrogen for 18h. The acetonitrile was evaporated in vacuo, the residue dissolved in dichloromethane (50ml) and washed with 2N sodium carbonate (20ml) and then 0.5N hydrochloric acid (10ml). The organic phase was dried and evaporated in vacuo, and the residue purified by flash chromatography eluting with dichloromethane-methanol (9:1) to give the title compound as a crystalline solid (0.80g) m.p. 108-110°.

Intermediate 2

1-[(3,4-Dichlorophenyl)acetyl]-2-piperidinecarboxaldehyde

Dry chromium trioxide (5.40g) was added portionwise to a solution of pyridine (9.0g, 8.6ml) in dry dichloromethane (120ml) at room temperature under nitrogen and the resulting solution was stirred for 30min. A solution of Intermediate 1 (3.0g) in dry dichloromethane (40ml) was added and the mixture stirred for 1h. The reaction mixture was poured into ether (2L) and filtered. The filtrate was evaporated to dryness and the residue purified by flash column chromatography eluting with ethyl acetate-hexane (1:1) to give the title compound as a crystalline solid (2.09g), m.p. 64-68°.

Intermediate 3

2-(1-Pyrrolidinylmethyl)piperidine maleate (1:2)

A mixture of 2-(1-pyrrolidinylmethyl)pyridine (4.0g) and platinum oxide (0.5g) in glacial acetic acid (100ml) was hydrogenated at 60 p.s.i./room temperature in a Cook hydrogenation apparatus for 18h. The catalyst was filtered off, the filtrate was evaporated and the residue was dissolved in dichloromethane (100ml). The organic solution was washed with 2N sodium hydroxide (2x50ml) and water (50ml) and dried ($MgSO_4$). The residue (3.7g) after evaporation was purified by chromatography on alumina (100g. Type UG1) eluting with dichloromethane-ethanol (95:5) to give the free base as an oil (1.95g). A portion of the free base (0.23g) was converted to the bismaleate salt (0.32g) in ether and crystallised from ethanol-ethyl acetate to give the title compound as microcrystals (0.30g), m.p. 168°.

Example 1

1-[(3,4-Dichlorophenyl)acetyl]-2-[(3-hydroxy-1-pyrrolidinyl)methyl] piperidine maleate (1:1)

A solution of Intermediate 2 (3.00g) in methanol (25ml) was added to a solution of 3-hydroxypyrrolidine (1.31g) in methanol (50ml) containing 5N hydrochloric acid (3.75ml) and 3Å molecular sieves (4g). Sodium cyanoborohydride (945mg) was added portionwise and the mixture stirred at room temperature for 3 days. The reaction mixture was filtered, the filtrate was concentrated in vacuo and the residue partitioned between dichloromethane (100ml) and aqueous 2N sodium carbonate solution (30ml). The aqueous layer was separated and re-extracted with dichloromethane (100ml). The combined organic extracts were washed with water, dried and evaporated to give a gum. This material was purified by flash column chromatography eluting with dichloromethane-methanol (9:1), to give the title compound as the free base (2.59g). A portion of the free base (511mg) was treated with maleic acid (160mg) and the resulting salt crystallised from ethyl acetate-ethanol to give the title compound as a solid (423mg), m.p. 141-144°.

| | | | | | | | | | |
|---|---|---|---|---|---|---|---|---|---|
| Analysis | Found: | C, | 54.28; | H, | 5.60; | N, | 5.63; | Cl, | 14,68. |
| $C_{18}H_{24}Cl_2N_2O_2.C_4H_4O_4$ | requires | C, | 54.22; | H, | 5.79; | N, | 5.75; | Cl, | 14.55%. |

Example 2

Methyl 1-[[1-[(3,4-dichlorophenyl)acetyl]-2-piperidinyl]methyl]-1,2,5,6-tetrahydro-3-pyridinecarboxylate fumarate (1:1)

A solution of Intermediate 2 (1.0g) in methanol (10ml) was added to a mixture of methyl 1,2,5,6-tetrahydro-3-pyridinecarboxylate (2.80g), 5N methanolic hydrogen chloride (1.3ml) and 3Å molecular sieves (2.0g). The reaction mixture was stirred for 10min before sodium cyanoborohydride (222mg) was added portionwise and the mixture was stirred at room temperature for 23h. The fine suspension was filtered off, the filtrate was evaporated and the residue was dissolved in dichloromethane (20ml) and washed with 2N sodium carbonate (10ml). The aqueous layer was further extracted with dichloromethane (2x10ml) and the combined organic extracts were dried, filtered and evaporated to afford an oil. The crude oil was purified by flash column

chromatography eluting with dichloromethane-methanol-aqueous ammonia 19:1:0.1 to give the title compound as the free base (962mg). The free base (962mg) was further purified by flash column chromatography on silica (deactivated by triethylamine) eluting with ethyl acetate - hexane 1:1 to give pure product (251mg) which was dissolved in ethyl acetate and treated with a solution of fumaric acid (75mg) in ethyl acetate/methanol. The solvent was removed and the resulting oil was triturated under dry ether to give the title compound as a crystalline solid (88mg), m.p. 70-80°C.

| | | | | | | | |
|---|---|---|---|---|---|---|---|
| Analysis | Found: | C, | 54.31; | H, | 5.52; | N, | 4.82. |
| $C_{21}H_{26}CL_2N_2O_3.C_4H_4O_4.2/3H_2O$ | requires | C, | 54.25; | H, | 5.71; | N, | 5.06. |

Example 3

1-[[1-[(3,4-Dichlorophenyl)acetyl]-2-piperidinyl]methyl]-1,2,5,6-tetrahydro-3-pyridinecarboxamide maleate

I) 1-[[1-[(3,4-Dichlorophenyl)acetyl]-2-piperidinyl]methyl]-1,2,5,6-tetrahydro-3-pyridinecarboxylic acid

Lithium hydroxide (0.04g) was added to a stirred solution of the free base of the product of Example 2 (0.2g) in a mixture of tetrahydrofuran (10ml) and water (10ml) and the mixture was stirred for 2h.
A further quantity of lithium hydroxide (0.02g) was added and the mixture was allowed to stand for 18h. The reaction mixture was neutralised with glacial acetic acid and the tetrahydrofuran was removed under reduced pressure. The precipitated solid was filtered off and dried to give the title compound as a solid (130mg) m.p. 195-6° (with decomp.).
T.l.c. (Silica) ethylacetate:isopropanol:water:0.880 ammonia (25:15:8:2), detection u.v., IPA, Rf 0.36.

II) 1-[[1-[(3,4-Dichlorophenyl)acetyl]-2-piperidinyl]methyl]-1,2,5,6-tetrahydro-3-pyridinecarboxamide maleate (1:1)

1,1′-Carbonyldiimidazole (219mg) was added to a stirred solution of the product of stage (I) (370mg) in dry dichloromethane (2ml). The resulting solution was stirred at room temperature for 1h whilst degassing with nitrogen. The solution was cooled to -40° and ammonia was condensed in via a dry ice condenser until the volume had doubled. The mixture was stirred at -40° for 2h and allowed to warm to room temperature overnight. The reaction mixture was diluted with dichloromethane (20ml) and washed with 2N sodium carbonate (2x20ml). The organic extract was dried ($MgSO_4$) and evaporated to give a gum which was purified by flash column chromatography eluting with dichloromethane:methanol:0.880 ammonia (180:8:1) to give the free base of the title compound as a foam (160mg). The free base (160mg) was dissolved in ethyl acetate and treated with a solution of maleic acid (50mg) in ethyl acetate. Diethyl ether was gradually added and the resulting gum was triturated to give the title compound as a solid (139mg) m.p. 170-173°.

| | | | | | | | |
|---|---|---|---|---|---|---|---|
| Analysis | Found: | C, | 54.30; | H, | 5.57; | N, | 7.79; |
| $C_{20}H_{25}Cl_2N_3O_2.C_4H_4O_4$ | requires | C, | 54.76; | H, | 5.55; | N, | 7.98%. |

Example 4

1-[(3,4-Dichlorophenyl)acetyl]-2-[1-(3-oxo-1-pyrrolidinyl)methyl]piperidine maleate (1:1)

Dimethylsulphoxide (0.925g) in dichloromethane (3ml) was added to a stirred solution of oxalyl chloride (0.753g) in dichloromethane (12ml) at -50° to -60°. The mixture was stirred for 3min and the product of Example 1 as the free base (2g) in dichloromethane (5ml) was added over a 5min period. Stirring was continued for an additional 15min and then triethylamine (2.72g) was added. The reaction mixture was stirred for 5min and then allowed to warm to room temperature. Water (30ml) was added, the organic layer was separated and the aqueous layer was further extracted with dichloromethane (30ml). The combined organic phases were washed with saturated aqueous sodium chloride solution, dried, and the solvent removed in vacuo to give an oil which crystallized upon standing at 4°. This material was crystallized from ethyl acetate-diethyl ether to give the free base of the title compound (0.605g). The mother liquors were purified by column chromatography on alumina (type UG1), eluting with ethyl acetate, to give a further quantity of product (0.615g). The maleate salt was formed in the conventional manner and recrystallised from ethylacetate - ethanol to give the title compound as a solid (422mg), m.p. 152-155°.

| | | | | | | | | | |
|---|---|---|---|---|---|---|---|---|---|
| Analysis | Found: | C, | 54.46; | H, | 5.41; | N, | 5.64; | Cl, | 14.80. |
| $C_{18}H_{22}Cl_2N_2O_2$ | requires | C, | 54.44; | H, | 5.40; | N, | 5.77; | Cl, | 14.61%. |

Example 5

1-[(3,4-Dichlorophenyl)acetyl]-2-[(3-methylenelpyrrolidinyl)methyl]piperidine maleate (1:1)

The product of Example 4 as the free base (0.61g) in dry dimethylsulphoxide (5ml) was added over a 10 min. period to the Wittig reagent prepared from sodium hydride (0.16g, 50% dispersion with oil) and methyltriphenylphosphonium bromide (1.18g) in dry dimethylsulphoxide (15ml, distilled over calcium hydride). The reaction mixture was stirred at 45° for 1h, cooled, and poured into ice-water. The resultant aqueous mixture was extracted with ethyl acetate (2x100ml) and the combined organic extracts were washed with saturated aqueous sodium chloride solution, dried and the solvent removed in vacuo to leave a gum. This material was purified by column chromatography on alumina (type UG1), eluting with diethyl ether-ethyl acetate (1:1), to give the free base of the title compound as a gum (500mg). Maleate salt formation followed by crystallisation from ethyl acetate - ethanol gave the title compound as a solid (383mg), m.p. 129-131°.

| | | | | | | | | | | | |
|---|---|---|---|---|---|---|---|---|---|---|---|
| Analysis | Found: | C, | 57.15; | H, | 5.72; | N, | 5.68; | Cl, | 14.44. |
| $C_{19}H_{24}Cl_2N_2O.C_4H_4O_4$ | requires | C, | 57.15; | H, | 5.84; | N, | 5.80; | Cl, | 14.67%. |

Example 6

2-[[3-(Cyanomethylene)-1-pyrrolidinyl]methyl]-1-[(3,4-dichlorophenyl) acetyl]piperidine hydrochloride

A solution of diethylcyanomethylphosphonate (1.06g) in dry tetrahydrofuran (10ml) was added dropwise to a stirred suspension of sodium hydride (0.27g) in dry tetrahydrofuran (80ml) at 0-5°C. Stirring was continued at 0-5° for 1h and a solution of the product of Example 4 as the free base (2g) in dry tetrahydrofuran (25ml) was added. The mixture was stirred at ambient temperature for 18h and heated at reflux for 1h. The reaction mixture was cooled, poured into aqueous sodium chloride (12% w/v; 50ml) and extracted with ethyl acetate (2x75ml). The organic extract was washed with brine (20ml), dried ($MgSO_4$) and evaporated in vacuo. The residue was purified by flash column chromatography eluting with ethyl acetate/ethanol (100:1) to give isomers of the free base as an oil (Isomer I 0.417g) and a solid (Isomer II 0.97g).

A portion of Isomer I (0.2g) was dissolved in diethyl ether (10ml) and treated with ethereal hydrogen chloride and the resulting solid was crystallized from ethyl acetate and methanol to give the hydrochloride salt of the title compound (Isomer I) as a solid (0.18g) m.p. 218-221°.

| | | | | | | | |
|---|---|---|---|---|---|---|---|
| Analysis | Found: | C, | 55.78; | H, | 5.63; | N, | 9.47. |
| $C_{20}H_{23}Cl_2N_3O.HCl$ | requires | C, | 56.02; | H, | 5.64; | N, | 9.80%. |

Isomer II (0.97g) was crystallised from methyl acetate/hexane to give the title compound (Isomer II) as a solid (0.2g), m.p. 88-90°.

| | | | | | | | |
|---|---|---|---|---|---|---|---|
| Analysis | Found: | C, | 61.07; | H, | 6.09; | N, | 10.59. |
| $C_{20}H_{23}Cl_2N_3O$ | requires | C, | 61.23; | H, | 5.91; | N, | 10.71%. |

Example 7

Methyl [1-[[1-[(3,4-dichlorophenyl)acetyl]-2-piperidinyl]methyl]-3-pyrrolidinylidene]acetate fumarate (1:1)

A solution of trimethyl phosphonoacetate (543mg) in dry tetrahydrofuran (2ml) was added dropwise to a stirred suspension of sodium hydride (50% dispersion, 153mg) in dry tetrahydrofuran (60ml) under nitrogen. The suspension was stirred for 5min and a solution of the product of Example 4 as the free base (1.0g) in dry tetrahydrofuran (5ml) was added dropwise. The reaction mixture was refluxed under nitrogen for 2.5h. The solution was cooled, quenched with water (60ml), and extracted with ethyl acetate (3x100ml). The combined organic extracts were washed with saturated sodium chloride solution, dried and evaporated to give an oil. Purification by column chromatography on alumina (Type UGI, diameter 2.5cm) eluting with ethyl acetate - hexane (1:1) gave the title compound as the free base as a mixture of isomers (782mg), slightly contaminated with starting material. Further purification by flash chromatography eluting with ethanol-ethyl acetate (1:49) gave an upper running component Isomer I (269mg) and a lower running component Isomer (II) (267mg).

A portion of isomer I (236mg) was dissolved in ether and treated with a solution of fumaric acid (71mg) in ether/methanol and the solution left to crystallise overnight at 4° to give the title compound (isomer I) as a crystalline solid (156mg) m.p. 161-163°. T.l.c. (Silica) ethanol - ethyl acetate (1:19), u.v. IPA, Rf 0.4.

The lower running Isomer II (262mg) was similarly converted into the fumarate salt to give the title compound (isomer II) as a crystalline solid (172mg) m.p. 115-118°.

TLC (silica) ethanol-ethyl acetate (1:19) u.v., IPA, Rf 0.3

Example 8

a) 1-[(3,4-Dichlorophenyl)acetyl]-2-[[3-(methoxyimino)-1-pyrrolidinyl]methyl] piperidine maleate (1:1)

Methoxyamine hydrochloride (272mg) was added to a solution of the product of Example 4 as the free base (1.0g) in dry pyridine (15ml), and the reaction mixture stirred at room temperature for 18h. The solvent was removed in vacuo and the residue was partitioned between dichloromethane (20ml) and 2N sodium hydroxide (20ml). The aqueous layer was further extracted with dichloromethane (2x20ml). The combined organic extracts were dried and evaporated to give a gum. Purification by flash column chromatography eluting with methanol-dichloromethane (1:19) gave the free base of the title compound as a mixture of isomers. Further purification by flash column chromatography eluting with ethanol-ethyl acetate (1:49) gave an upper running isomer I (364mg) and a lower running isomer II (282mg).

Isomer I (359mg) was dissolved in ethyl acetate and treated with a solution of maleic acid (116mg) in ethyl acetate. A small amount of hexane was added and the solution was allowed to crystallise overnight to give the title compound (isomer I) as a crystalline solid (235mg) m.p. 100-103°.

T.l.c. (Silica) ethanol-ethyl acetate (1:19), u.v., IPA, Rf 0.54

b) 1-[(3,4-Dichlorophenyl)acetyl]-2-[[3-(methoxyimino)-1-pyrrolidinyl]methyl]piperidine fumarate (1:1)

Isomer (II) (244mg) obtained by the method of part (a) above was dissolved in ether and treated with a solution of fumaric acid (78mg) in ether-methanol. A small amount of hexane was added and the salt was allowed to crystallise over 18h to give the title compound (Isomer II) as a crystalline solid (137mg), m.p. 150-153°.

T.l.c. (silica) ethanol-ethylacetate (1:19), u.v., IPA, Rf 0.47.

Example 9

1-[(3,4-Dichlorophenyl)acetyl]-2-(2-isoxazolidinylmethyl)piperidine hydrochloride

Sodium cyanoborohydride (0.38g) was added to a stirred mixture of isoxazolidine hydrochloride (0.44g), Intermediate 2 (0.60g), 3Å molecular sieves (1.2g) and triethylamine (about 0.5-1ml) in methanol (20ml) at pH6 under nitrogen. The reaction mixture was stirred at room temperature for 24h, filtered and concentrated in vacuo. The residue was basified with 2N sodium hydroxide solution (20ml) and extracted with dichloromethane (3x20ml). The combined extracts were dried, concentrated in vacuo and purified by flash column chromatography eluting with dichloromethane:methanol:0.88 ammonia (300:6:1) to give the free base of the title compound as a gum (370mg). Ethereal hydrogen chloride was added to a solution of the free base in diethyl ether and the resulting solid was triturated under diethyl ether to give the title compound as an amorphous solid (350mg) m.p. 170-172°C.

| | | | | | | | | | |
|---|---|---|---|---|---|---|---|---|---|
| Analysis | Found: | C, | 51.50; | H, | 5.89; | N, | 6.89; | Cl, | 27.20. |
| $C_{17}H_{22}Cl_2N_2O_2.HCl$ | requires | C, | 51.86; | H, | 5.89; | N, | 7.11; | Cl, | 27.01%. |

Example 10

1-(2-(Naphthalenylacetyl)-2-(1-pyrrolidinylmethyl)piperidine maleate (1:1)

To a solution of 2-naphthylacetic acid (386mg) in acetonitrile (10ml) was added a solution of 1,1′-carbonyldiimidazole (337mg) in warm acetonitrile (5ml). After 10min a solution of Intermediate 3 as the free base (0.35g) in acetonitrile (5ml) was added and the mixture was stirred at room temperature for 4h. The solvent was removed in vacuo and the residue partitioned between dichloromethane (50ml) and 2N aqueous sodium carbonate solution (50ml). The layers were separated and the aqueous layer was further extracted with dichloromethane (25ml). The combined organic extracts were washed with water, dried and the solvent removed in vacuo to give a gum. Purification by chromatography on alumina (type UGI), eluting with ethyl acetate, gave the free base of the title compound (0.47g) as a gum which was converted to the maleate salt by conventional methods and crystallised from ethyl acetate - ethanol to give the title compound as a solid (0.37g) m.p. 144-146°.

| | | | | | | | |
|---|---|---|---|---|---|---|---|
| Analysis | Found: | C, | 69.23; | H, | 7.23; | N, | 6.17. |
| $C_{22}H_{28}N_2O.C_4H_4O_4$ | requires | C, | 69.0; | H, | 7.1; | N, | 6.2%. |

Example 11

1-(1-Naphthalenylacetyl)-2-(1-pyrrolidinylmethyl)piperidine maleate (1:1)

1,1′-Carbonyldiimidazole (0.96g) was added to a stirred solution of 1-naphthylacetic acid (1.0g) in dichloromethane (50ml) through which was passed a steady stream of nitrogen. A solution of Intermediate 3

as the free base (0.91g) in dichloromethane (25ml) was added and the mixture was kept at room temperature for 18h. The reaction mixture was washed with 8% sodium bicarbonate solution (30ml), water (30ml), dried ($MgSO_4$) and evaporated to give an oil. The oil was purified by column chromatography on alumina (Grade II) eluting with ethyl acetate to give the free base as an oil (1.4g). A solution of the oil in ethyl acetate (50ml) was treated with a solution of maleic acid (0.49g) in ethyl acetate (15ml) and the mixture was stirred for 30min. The precipitated solid was filtered off and dried to give the title compound as a solid (1.7g) m.p. 170.5-172°.

| | | | | | | | |
|---|---|---|---|---|---|---|---|
| Assay | Found: | C, | 68.65; | H, | 7.1; | N, | 6.0; |
| $C_{22}H_{28}N_2O.C_4H_4O_4$ | requires | C, | 69.0; | H, | 7.1; | N, | 6.2% |

Example 12

1-[(3-,4-Dichlorophenyl)acetyl]-2-[(4-oxo-1-piperidinyl)methyl] piperidine maleate (1:1)

(i) 1-[(3,4-Dichlorophenyl)acetyl]-2-[(1,4-dioxa-8-azaspiro [4,5]dec-8-yl)methyl]piperidine maleate (1:1)

The title compound was obtained as a solid (315mg) from Intermediate 2 (1.02g) and 1,4-dioxa-azaspiro (4.5)decane (2.92g) by the method of Example 9 after purification by flash column chromatography eluting with ether:methanol (9:1) and maleate salt formation, m.p. 172.5-174°.

| | | | | | | | |
|---|---|---|---|---|---|---|---|
| Analysis | Found: | C, | 55.30; | H, | 6.10; | N, | 5.05. |
| $C_{21}H_{28}Cl_2N_2O_3.C_4H_4O_4$ | | C, | 55.25; | H, | 5.95; | N, | 5.15%. |

(ii) 1-[(3,4-Dichlorophenyl)acetyl]-2-[(4-oxo-1-piperidinyl)methyl] piperidine maleate (1:1)

Dilute sulphuric acid (1N, 10ml) was added to a solution of the product of Stage (i) (630mg) in acetone (20ml) and the mixture heated under reflux for a total of 88h. After cooling to room temperature the acetone was removed in vacuo and the residue basified with aqueous sodium carbonate (2N, 4ml) and saturated aqueous sodium bicarbonate (ca 4ml). The mixture was extracted with ethyl acetate (3x10ml) and the combined extracts dried and evaporated giving an oil (448mg). This was purified by flash column chromatography eluting with ether-methanol (9:1) to give pure title compound as base (380mg) as a solid m.p. 106-108°. The solid was dissolved in ether (18ml)/methanol (2ml) and the solution added to a solution of maleic acid (107mg) in ether (10ml) giving on scratching a solid. Heating with isopropanol (75ml) and allowing to cool to room temperature left a small amount of insoluble solid which was filtered off. Evaporation of the filtrate gave pure title compound (290mg) as a solid, m.p. 179-181°. dec.

| | | | | | | | |
|---|---|---|---|---|---|---|---|
| Analysis Found | | C: | 55.45; | H, | 5.75; | N, | 5.35 |
| $C_{19}H_{24}Cl_2N_2O_2.C_4H_4O_4$ 0.2$C_3H_8O$ | Requires | C: | 55.45; | H, | 5.85; | N, | 5.5% |

Example 13

1-[(3,4-Dichlorophenyl)acetyl]-2-[(4-methylene-1-piperidinyl)methyl] piperidine maleate (1:1)

Sodium hydride (1.2g of a 50% dispersion in oil, 25mmol) was added to dry dimethyl sulphoxide (10ml) under nitrogen and the mixture stirred at 60° until hydrogen evolution ceased. After cooling to room temperature a solution of methyltriphenylphosphonium bromide (8.93g) in dry dimethylsulphoxide (25ml) was added and the mixture stirred under nitrogen for 10min. A solution of the free base of the product of Example 12 (960mg) in dry dimethylsulphoxide (20ml) was added and the mixture stirred under nitrogen at 60° for 20h. The reaction was then quenched by being poured into saturated aqueous ammonium chloride solution (100ml) and the product extracted into ether (3x100ml). The ether extracts were combined and extracted with dilute hydrochloric acid (0.1M, 3x20ml). The combined acid extracts were washed with ether (100ml), basified with aqueous sodium carbonate (1M, 8ml), and extracted with ether (3x40ml). These ether extracts were combined, washed with saturated aqueous sodium chloride, dried and evaporated giving crude title compound free base as an oil (1.64g). This was purified by flash column chromatography eluting with ether-methanol (19:1) to give pure title compound free base (830mg) as an oil. T.l.c. Silica/Ether-methanol (19:1) Rf 0.43. The free base was converted into the maleate salt by conventional methods, m.p. 136-138

| | | | | | | | |
|---|---|---|---|---|---|---|---|
| Analysis Found | : | C, | 58.05; | H, | 6.05; | N, | 5.4 |
| $C_{20}H_{26}Cl_2N_2O.C_4H_4O_4$ | requires : | C, | 57.95; | H, | 6.10; | N, | 5.65 |

Example 14

2-[(3-Hydroxy-1-pyrrolidinyl)methyl]-1-[[4-(trifluoromethyl)phenyl]-acetyl]piperidine fumarate (1:1) salt

(i) 1-(2-Pyridinylmethyl)-3-pyrrolidinol

A mixture of 3-pyrrolidinol (15g) and triethylamine (72mℓ) in water (100mℓ) at 0 to 5° was treated with 2-picolinylchloride hydrochloride (27g). The mixture was stirred at ambient temperature for 4h and poured into aqueous sodium carbonate (2M; 300mℓ). The mixture was extracted with dichloromethane (3x250mℓ) and the aqueous solution was saturated with sodium chloride, filtered and extracted wtih dichloromethane (2x100mℓ). The combined organic extracts were dried, and evaporated leaving an oily residue (26g) which was purified by distillation (b.p. 138°/ 0.6mmHg) to give the title compound as an oil (19g).
T.l.c. $SiO_2$ (Dichloromethane/methanol/ammonia 75:10:2), Rf 0.55.

(ii) 1-(2-Piperidinylmethyl)-3-pyrrolidinol

A solution of the product of stage (i) (19g) in glacial acetic acid (300mℓ) was hydrogenated over Adam's catalyst (1.5g) at room temperature for 20h. The mixture was filtered and the filtrate was evaporated in vacuo. The residue was dissolved in aqueous sodium hydroxide (5M; 300mℓ) and was extracted with chloroform (2x300mℓ). The organic extract was washed with aqueous sodium hydroxide (5M; 50mℓ), dried and evaporated in vacuo. The residue (20g) was purified by distillation to give the title compound as an oil (15g) (b.p. 112-114° 0.5mmHg).
T.l.c. ($SiO_2$ Dichloromethane/methanol/ammonia 75:10:2), Rf 0.1.

(iii) 2-[(3-Hydroxy-1-pyrrolidinyl)methyl]-1-[[4-(trifluoromethyl)phenyl]acetyl]piperidine fumarate (1:1) salt

1'1,Carbonyldiimidazole (528mg) was added to a stirred solution of (α,α,α-trifluoro-p-tolyl)acetic acid (554mg) in dry dichloromethane (20mℓ) and the resulting solution was stirred at room temperature under nitrogen for 2h. The solution was added dropwise to a solution of the product of stage (ii) (500mg) in dichloromethane (20mℓ) and the resultant solution stirred for 2 days. The solution was poured into sodium carbonate solution (2N, 100mℓ), the layers separated and the aqueous extracted with dichloromethane (3x100mℓ). The combined extracts were dried and evaporated to give an oil (1.34g). The product was purified by flash column chromatography eluting with dichloromethane:methanol: ammonia, 140:8:1 changing to 125:8:1 to give the free base of the title compound as an oil (600mg). The oil (597mg, 1.6mmol) in ethyl acetate (10mℓ) was treated with fumaric acid (195mg, 1.7mmol) in ethyl acetate:methanol (1:1, ~10mℓ). The salt was filtered off to give the title compound as a powder (520mg) m.p. 168-172°.

| | | | | | | | | |
|---|---|---|---|---|---|---|---|---|
| Analysis found | | C, | 56.05; | H, | 6.2; | N, | 5.7 |
| $C_{19}H_{25}F_3N_2O_2.0.82C_4H_4O_2.0.6H_2O$ | requires | C, | 56.2; | H, | 6.2; | N, | 5.9% |

Example 15

1-(3,4-Dichlorophenylacetyl)-2-[1-(2-hydroxymethylpyrrolidinyl) methyl]piperidine maleate salt

Sodium cyanoborhydride (627mg, 9.95mmol) was added portionwise to a solution of Intermediate 2 (1.98g) in methanol (50ml) to which had previously been added : L-2-pyrrolidinemethanol (1.00g), methanolic hydrogen chloride (5N, 2.5ml) and 3Å molecular sieves (2.5g). The reaction mixture was stirred at room temperature for 5 days, filtered and the filtrate was concentrated in vacuo. The residue was partitioned between dichloromethane (400ml) and aqueous sodium carbonate (2N, 200ml). The aqueous layer was separated and re-extracted with dichloromethane (200ml). The combined organic extracts were dried and evaporated in vacuo to give a gum (~2.6g) which was purified by flash column chromatography eluting with dichloromethane-methanol (9:1) to give the title compound as a gum (761mg) (ISOMER I). Further elution gave a mixture of isomers I and II (305mg) and then isomer II as a gum (814mg).
T.l.c. $SiO_2$ $CH_2Cl_2$-MeOH (9:1), Rf 0.32 (ISOMER I) ; Rf 0.20 (ISOMER II). Isomer I was transformed into the maleate salt, m.p. 109-110°

| | | | | | | | | |
|---|---|---|---|---|---|---|---|---|
| Analysis Found | | C, | 54.75; | H, | 6.03; | N, | 5.41 |
| $C_{19}H_{26}Cl_2N_2O_2.C_4H_4O_4$ | requires | C, | 55.09; | H, | 6.03; | N, | 5.59% |

Example 16

1-[(3,4-Dichlorophenyl)acetyl]-2-[(2-ethenyl-1-pyrrolidinyl)methyl] piperidine maleate

(i) 2-ethenylpyrrolidine hydrochloride salt

Vinyl chloroformate (1.40mℓ) was added dropwise to a stirred mixture of 2-ethenyl-1-(triphenylmethyl)-2-pyrrolidine (2.85g) and potassium carbonate (0.5g) in dry dichloromethane (50mℓ) at -30° under nitrogen. The resulting mixture was refluxed under nitrogen for 3.5h, then the solvent evaporated in vacuo and the residue was purified by flash column chromatography eluting with 15% ethyl acetate - hexane to give the intermediate carbamate as a crystalline solid (3.2g contaminated with triphenylmethyl impurities). The carbamate (3.1g) was dissolved in a mixture of methanol (50mℓ) and ethereal HCl (30mℓ). The resulting solution was refluxed for 2h under nitrogen, then the ether was distilled off. The reaction mixture was refluxed for a further 1h. The solvent was evaporated in vacuo and the residue was triturated with dry ether to give the title compound as a crystalline solid (8.79mg).

T.l.c. 10% MeOH - $CH_2Cl_2$ Rf 0.07.

(ii) 1-[(3,4-Dichlorophenyl)acetyl]-2-[(2-ethenyl-1-pyrrolidinyl) methyl]piperidine fumarate (1:1)

A solution of intermediate 2 (1.0g) in methanol (50mℓ) was added with stirring to a mixture of 2-ethenylpyrrolidine hydrochloride salt (871mg, 6.5mmol), triethylamine (0.7mℓ, 5.0mmol) and 3Å molecular sieves (2.0g) in methanol (5mℓ) under nitrogen. The reaction mixture was stirred for 10 min, before sodiumcyanoborohydride (232mg) was added portionwise and the mixture stirred at room temperature for 36h. The fine suspension was filtered off and the filtrate evaporated. The residue was dissolved in dichloromethane (20mℓ) and washed with 2N sodium carbonate (10mℓ). The aqueous layer was further extracted with dichloromethane (2x10mℓ). The combined organic extracts were dried, and evaporated to give an oil. The crude oil was purified by column chromatography on alumina (UGI, diameter 3 cm, length 15cm) : eluting with ethyl acetate - hexane 1:3 to give the free base as a mixture of isomers (547mg). The free base was re-purified by flash column chromatography eluting with 3% MeOH -$CH_2Cl_2$ to give the free base as a mixture of isomers. A portion of the free base (215mg) in ether was treated with a solution of fumaric acid (76mg) in ether/methanol. The solution was allowed to crystallise overnight at 0°. The solvent was decanted off and the crystals washed with a small amount of dry ether, and dried to give the title compound as a cream crystalline solid. (175mg). (m.p. 147-150°).

(iii) 1-[(3,4-Dichlorophenyl)acetyl]2-[(2-ethenyl-1-pyrrolidinyl) methyl]piperidine maleate

The mixture of isomers of 1-[(3,4-Dichlorophenyl)acetyl] 2-[(2-ethenyl-1-pyrrolidinyl)methyl]piperidine were separated by HPLC to give the title compound, (isomer 1) as an oil (102mg) and isomer 2 as a crystalline solid (44mg). The free base of isomer 1 (102mg) in ether was treated with a solution of maleic acid (34mg) in ether. The resultant gum was triturated to give a solid. The solvent was decanted off and the solid dried to give the title compound as a solid (71mg) (m.p. 113-115°).

| | | | | | | | |
|---|---|---|---|---|---|---|---|
| Analysis | Found : | C, | 57.83; | H, | 6.19; | N, | 5.56%. |
| $C_{20}H_{26}Cl_2N_2O.C_4H_4O_4$ | requires | C, | 57.95; | H, | 6.08; | N, | 5.63%. |

Isomer 2 m.p. 96-98°.

| | | | | | | | |
|---|---|---|---|---|---|---|---|
| Analysis | Found : | C, | 62.90; | H, | 7.00; | N, | 7.23%. |
| $C_{20}H_{26}Cl_2N_2O$ | requires | C, | 62.99; | H, | 6.87; | N, | 7.35%. |

Example 17

1-[(3,4-Dichlorophenyl)acetyl]-2-[[3-(3-phenylpropylidene)-1-pyrrolidinyl] methyl]piperidine maleate

To a stirred suspension of 3-phenylpropyltriphenylphosphonium bromide (2.49g) in dry tetrahydrofuran (30ml) at -60° under nitrogen, was added a solution of n-butyllithium in hexane (1.6M, 0.3ml) until a permanent yellow colour had formed. Further n-butyllithium (3.4ml, 5.4mmol) was added dropwise. The reaction mixture was warmed to 0° and stirring continued for 1h. A solution of the product of Example 4 in dry tetrahydrofuran (5ml) was added dropwise at -60°, and the reaction mixture allowed to warm to room temperature. Stirring was continued overnight. The reaction mixture was quenched with water (20ml) and the layers separated. The organic layer was evaporated in vacuo and the residue partitioned between ether (10ml) and water (10ml). The aqueous layer was further extracted with ether (3x10ml). The combined organic extracts were dried and evaporated to give an oil which was purified by column chromatography on alumina (type UGI, diameter 2.5cm) eluting with ether-hexane 4:1, followed by 2:1 ether-hexane to 1:1 ether-hexane to ethyl acetate to give the free base of the title compound. The free base (235mg) was dissolved in ether and treated with a solution of maleic acid (64mg) in ether. The oil was triturated with ether to give a solid. The solvent was decanted off, the crystals washed with a small amount of ether, and dried to give the title compound as a crystalline solid (148mg) m.p. 98-100°.

| | | | | | | | |
|---|---|---|---|---|---|---|---|
| Analysis Found | | C, | 63.02; | H, | 5.99; | N, | 4.62 |
| $C_{27}H_{32}N_2OCl_2.C_4H_4O_4$ | requires | C, | 63.37; | H, | 6.18; | N, | 4.77% |

Example 18

[1-[[1-(3,4-Dichlorophenyl)acetyl]-2-piperidinyl]methyl]-3-pyrrolidinylidene]acetic acid

A solution of the product of Example 7 (Isomer I) (0.34g) in a mixture of tetrahydrofuran (3mℓ) and water (3mℓ) was treated with lithium hydroxide (56mg; 1.3mmol) and the mixture was stirred at ambient temperature for 24h. The mixture was neutralised with acetic acid (pH 6.5) and the organic solvent was evaporated in vacuo.

Crystallisation of the resulting precipitate from ethyl acetate/methanol to give the title compound (Isomer I) as a solid (0.12g) m.p. 53.5° softens.

T.l.c. ($SiO_2$ Dichloromethane/methanol/ammonia 75:10:2) Rf 0.05.

| | | | | | | | |
|---|---|---|---|---|---|---|---|
| Assay | Found: | C, | 57.41; | H, | 6.2; | N, | 6.16. |
| $C_{20}H_{24}Cl_2N_2O_3.0.5H_2O$ | requires | C, | 57.15; | H, | 6.0; | N, | 6.67%. |

G.l.c. Isomer ratio 85:15 Isomer I:Isomer II Crystallisation from t-butylmethylether and hexane gave the title compound (Isomer 2) as a solid (80mg) m.p. 81-3° softens.

| | | | | | | | |
|---|---|---|---|---|---|---|---|
| Assay | Found: | C, | 57.9; | H, | 5.7; | N, | 6.50. |
| $C_{20}H_{24}Cl_2N_2O_3$ | requires | C, | 58.4; | H, | 5.9; | N, | 6.8%. |

T.l.c. Silica/dichloromethane/methanol/ammonia 75:10:2 Rf 0.1.

Example 19

2-[1-[[1-[(3,4-Dichlorophenyl)acetyl]-2-piperidinyl]methyl]-3-pyrrolidinylidene]acetamide hydrochloride

A solution of the product of Example 18 (Isomer I) (0.42g) in dry dichloromethane (5mℓ) was treated with a solution of hydrogen chloride in diethyl ether (2mℓ) and the solvent was removed in vacuo. The solid residue was dissolved in dry dichloromethane (5mℓ) and treated with oxalyl chloride (0.135mℓ; 1.54mmol) followed by dimethyl formamide (1 drop). The mixture was stirred at ambient temperature for 1 h and was then added to aqueous ammonia (0.88; 5mℓ). The mixture was vigorously stirred for 1h and extracted with dichloromethane (2x20mℓ). The organic extract was washed with aqueous sodium carbonate (2M; 10mℓ) dried and evaporated leaving a foam (0.4g). The residue was purified by flash column chromatogrpahy eluting with dichloromethane/methanol/ammonia 150:8:1 to give the free base of the title compound as an oil (0.25g). This oil was dissolved in methylacetate (5mℓ) and treated with ethereal hydrochloride (0.5mℓ). The resulting solid was crystallized from ethyl acetate/methanol to give the title compound (Isomer I) as a solid (0.12g) m.p. 213°.

T.l.c. ($SiO_2$ Dichloromethane/methanol/ammonia 75:10:2) Rf 0.5.

| | | | | | | | |
|---|---|---|---|---|---|---|---|
| Analysis Found | | C, | 51.36; | H, | 6.30; | N, | 8.72 |
| $C_{20}H_{25}Cl_2N_3O.HCl.H_2O.\frac{1}{2}CH_3OH$ | requires | C, | 51.36; | H, | 6.22; | N, | 8.84 |

The product of Example 18 (Isomer II) was similarly reacted to give the title compound (Isomer II) as a solid (0.1g) on crystallisation from methanol/ethyl acetate m.p. 236°.

T.l.c. ($SiO_2$ Dichloromethane/methanol/ammonia 75:10:2) Rf 0.45.

| | | | | | | | |
|---|---|---|---|---|---|---|---|
| Analysis | Found: | C, | 51.47; | H, | 5.87; | N, | 8.92. |
| $C_{20}H_{25}Cl_2N_3O_2.HCl.H_2O$ | | C, | 51.68; | H, | 6.07; | N, | 9.04. |

Example 20

5-[(3,4-Dichlorophenyl)acetyl]-1,2,3,4-tetrahydro-1-[(3-hydroxy-1-pyrrolidinyl)methyl)]isoquinoline fumarate (1:1)

(i) 1-[(1,2,3,4-Tetrahydro-1-isoquinolinyl)carbonyl]-3-pyrrolidinol

A mixture of ethyl 1,2,3,4-tetrahydro-1-isoquinolinecarboxylate (1.0g) and 3-pyrrolidinol (1.25mℓ) was heated in a reactivial at 120° for ~4h. The excess pyrrolidinol was removed in vacuo and the residue was purified by flash column chromatography eluting with dichloromethane:methanol: ammonia (150:10:2 to 100:10:2) to give the title compound as a solid (800mg) m.p. 151-158°.

| | | | | | | | | |
|---|---|---|---|---|---|---|---|---|
| Analysis | Found: | C, | 68.2; | H, | 7.6; | N, | 11.2. |
| $C_{14}H_{18}N_2O_2$ | requires | C, | 68.3; | H, | 7.4; | N, | 11.4%. |

(ii) 1-[(1,2,3,4-Tetrahydro-1-isoquinolinyl)methyl]-3-pyrrolidinol

A suspension of lithium aluminium hydride (0.26g) in dry tetrahydrofuran (15mℓ) was treated with a suspension of the product of stage (i) (0.76g) in dry tetrahydrofuran (25mℓ) over 5min and the mixture was stirred at 40° for 2h. Water (0.25mℓ) was cautiously added, followed by aqueous sodium hydroxide solution (2N, 0.74mℓ) and water (0.25mℓ). The mixture was filtered and the filtrate was evaporated in vacuo to give a gum (560mg). The gum was purified by flash column chromatography eluting with dichloromethane:methanol: ammonia (150:10:1 to 100:10:1) to give the title compound as a gum (205mg).
T.l.c. $SiO_2$ ($CH_2Cl_2$:$CH_3OH$:$NH_3$, 75:10:2) Rf 0.35.

(iii) 5-[(3,4-Dichlorophenyl)acetyl]-1,2,3,4-tetrahydro-1-[(3-hydroxy-1-pyrrolidinyl)methyl]isoquinoline fumarate 1:1

1,1-Carbonyldiimidazole (150mg) was added to a solution of 3,4-dichlorophenylacetic acid (190mg) in dry dichloromethane (30mℓ), and the resulting mixture was stirred at room temperature for 25min. The mixture was added dropwise to a solution of the product of stage (ii) (205mg) and stirring was continued at room temperature for 18h. The reaction mixture was washed with 2N sodium carbonate solution (3x10mℓ) and the aqueous solution was extracted with dichloromethane (10mℓ). The organic layer was dried ($Na_2SO_4$) and evaporated to give a gum which was purified by flash column chromatography eluting with dichloromethane:methanol:ammonia (125:8:1) to give a gum (174mg). A solution of the gum (174mg) in ethyl acetate (10mℓ) was treated with a solutionof fumaric acid (50.7mg) in a mixture of hot methanol:ethyl acetate (10mℓ) to give the title compound as a solid (160mg) m.p. 194-198° decomp.

| | | | | | | | |
|---|---|---|---|---|---|---|---|
| Analysis | Found: | C, | 57.5; | H, | 5.25; | N, | 5.0. |
| $C_{22}H_{24}Cl_2N_2O_2.C_4H_4O_4.0.19H_2O$ | requires | C, | 57.95; | H, | 5.3; | N, | 5.2%. |

The following Examples illustrate pharmaceutical formulations containing 1-[(3,4-dichlorophenyl)acetyl]--2-[(3-oxo-1-pyrrolidinyl) methyl]-piperidine as active ingredient. Other compounds of formula (I) may be formulated in a similar manner.

TABLETS FOR ORAL ADMINISTRATION

DIRECT COMPRESSION

| | mg/tablet |
|---|---|
| Active ingredient | 20 |
| Calcium Hydrogen Phosphate B.P. * | 75.5 |
| Croscarmellose sodium USP | 4 |
| Magnesium Stearate, B.P. | 0.5 |
| Compression weight | 100mg |

* of a grade suitable for direct compression

The active ingredient is sieved before use. The calcium hydrogen phosphate, croscarmellose sodium and active ingredient are weighed into a clean polythene bag. The powders are mixed by vigorous shaking then the magnesium stearate is weighed and added to the mix which is blended further. The mix is then compressed using a Manesty F3 tablet machine fitted with 5.5mm flat bevelled edge punches, into tablets with target compression weight of 100mg.

Tablets may also be prepared by other conventional methods such as wet granulation.

Tablets of other strengths may be prepared by altering the ratio of active ingredient to lactose or the compression weight and using punches to suit.

The tablets may be film coated with suitable film forming materials, such as hydroxypropyl methylcellulose, using standard techniques. Alternatively the tablets may be sugar coated.

INJECTION FOR INTRAVENOUS ADMINISTRATION

| | mg/ml |
|---|---|
| Active ingredient | 5 |
| Sodium Chloride BP | as required |
| Water for Injection BP 0.5 to 2mℓ | |

Sodium chloride may be added to adjust the tonicity of the solution and the pH may be adjusted, using acid or alkali, to that of optimum stability and/or to facilitate solution of the active ingredient. Alternatively suitable buffer salts may be used.

The solution is prepared, clarified and filled into appropriate size ampoules sealed by fusion of the glass. The injection is sterilised by heating in an autoclave using one of the acceptable cycles. Alternatively the solution may be sterilised by filtration and filled into sterile ampoules under aseptic conditions. The solution may be packed under an inert atmosphere of nitrogen or other suitable gas.

INTRAVENOUS INFUSION

| | mg/ml |
|---|---|
| Dextrose 5% aqueous solution BP | 10-100ml |
| Active ingredient | 700mg |
| Sodium Chloride BP | as required |

For infusion at a rate of 700mg per hour.

## Claims

1. A compound of formula (I)

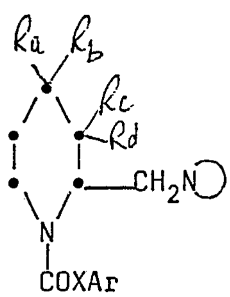

(I)

wherein $R_a$, $R_b$, $R_c$ and $R_d$ represent hydrogen atoms or $R_a$ and $R_c$ together form a bond and $R_b$ and $R_d$ together form -CH=CH-CH=CH-; -N◯ represents a 5-membered (optionally containing an oxygen atom adjacent to the nitrogen) or a 6-membered ring, which ring optionally contains one unit of unsaturation and which is unsubstituted or substituted by hydroxy, hydroxymethyl, oxo, optionally substituted methylidene, $-COR_1$ (where $R_1$ is $C_{1-6}$ alkyl, $-OR_2$ or $-NHR_2$, and $R_2$ is hydrogen, $C_{1-6}$ alkyl, aryl or ar($C_{1-6}$)alkyl) or $=NOR_3$ (where $R_3$ is $C_{1-6}$ alkyl);
X represents a direct bond, $-CH_2-$ or $-CH_2O-$;
Ar represents a substituted phenyl moiety (provided that when -N◯ does not contain an oxygen atom and is unsubstituted, Ar contains more than one unsaturated ring);
and physiologically acceptable salts thereof.

2. A compound according to Claim 1 wherein $R_a$, $R_b$, $R_c$ and $R_d$ represent hydrogen atoms.

3. A compound according to Claim 1 wherein $R_a$ and $R_b$ form a bond and $R_b$ and $R_d$ together form -CH=CH-CH=CH.

4. A compound according to any of Claims 1 to 3 wherein X represents $-CH_2-$.

5. A compound according to any of Claims 1 to 4 wherein Ar represents halosubstituted phenyl.

6. A compound according to any of Claims 1 to 5 wherein -N◯ represents a pyrrolidine ring which is unsubstituted or substituted by -OH, =O, =CHCN or =$CHCO_2CH_3$.

7. A compound according to Claim 1 wherein $R_a$, $R_b$, $R_c$ and $R_d$ represent hydrogen atoms; -N◯ represents a pyrrolidine ring substituted by =O, =CHCN or =$CHCO_2CH_3$; X represents -$CH_2$-; and Ar represents halosubstituted phenyl; and physiologically acceptable salts or solvates thereof.

8. A compound according to Claim 1 wherein $R_a$ and $R_c$ represent hydrogen atoms and $R_b$ and $R_d$ together form -CH=CH-CH=CH-; -N◯ represents a pyrrolidine ring substituted by -OH; X represents -$CH_2$-; and Ar represents halosubstiuted phenyl; and physiologically acceptable salts or solvates thereof.

9. A compound according to any of Claims 1 to 8 for use in therapy.

10. The use of a compound according to any of Claims 1 to 8 in the manufacture of a medicament for use in the treatment of pain and cerebral ischaemia.

11. A pharmaceutical composition which comprises a compound of formula (I) as defined in Claim 1 or a physiologically acceptable salt thereof together with a pharmaceutically acceptable carrier therefor.

## Claims/Process

1. A method for the preparation of compound of formula (I)

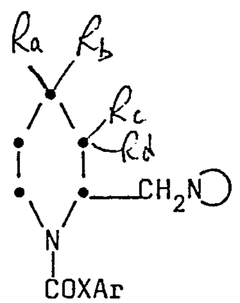

(I)

wherein

$R_a$, $R_b$, $R_c$ and $R_d$ represent hydrogen atoms or $R_a$ and $R_c$ together form a bond and $R_b$ and $R_d$ together form -CH=CH-CH=CH-; -N◯ represents a 5-membered (optionally containing an oxygen atom adjacent to the nitrogen) or a 6-membered ring, which ring optionally contains one unit of unsaturation and which is unsubstituted or substituted by hydroxy, hydroxymethyl, oxo, optionally substituted methylidene, -$COR_1$ (where $R_1$ is $C_{1-6}$ alkyl, -$OR_2$ or -$NHR_2$, and $R_2$ is hydrogen, $C_{1-6}$ alkyl, aryl or ar($C_{1-6}$)alkyl) or =$NOR_3$ (where $R_3$ is $C_{1-6}$ alkyl);

X represents a direct bond, -$CH_2$- or -$CH_2O$-;

Ar represents a substituted phenyl moiety (provided that when -N◯ does not contain an oxygen atom and is unsubstituted, Ar contains more than one unsaturated ring);

and physiologically acceptable salts thereof,

which method comprises :-

(A) reacting a compound of formula (II)

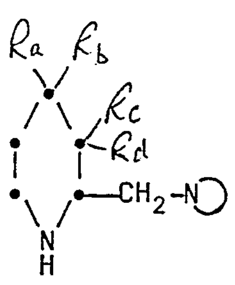

(II)

(wherein $R_a$, $R_b$, $R_c$, $R_d$ and -N◯ is as defined in Claim 1) with an acid $ArXCO_2H$ (wherein Ar and X are as defined in Claim 1) or an acylating agent corresponding thereto or a salt thereof;

or

(B) reductive amination of a compound of formula (V)

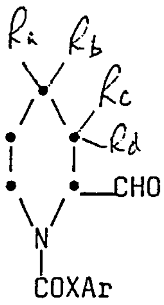

(V)

(wherein $R_a$, $R_b$, $R_c$, $R_d$, X and Ar are as defined in Claim 1) with an amine H-N (where -N is as defined in Claim 1) in the presence of a suitable reducing agent;

or

(C) subjecting a compound of formula (I) to an interconversion reaction and if necessary or desired subjecting the compound resulting from any of steps (A), (B) or (C) to one or two further reactions comprising :

(i) converting a compound of formula (I) or a salt thereof into a physiologically acceptable salt or solvate thereof

(ii) reduction of a racemic mixture to give a specific enantiomer.

2. A method according to Claim 1 wherein $R_a$, $R_b$, $R_c$ and $R_d$ represent hydrogen atoms.

3. A method according to Claim 1 wherein $R_a$ and $R_b$ form a bond and $R_b$ and $R_d$ together form -CH=CH-CH=CH.

4. A method according to any of Claims 1 to 3 wherein X represents $-CH_2-$.

5. A method according to any of Claims 1 to 4 wherein Ar represents halosubstituted phenyl.

6. A method according to any of Claims 1 to 5 wherein -N represents a pyrrolidine ring which is unsubstituted or substituted by -OH, =O, =CHCN or $=CHCO_2CH_3$.

7. A method according to Claim 1 wherein $R_a$, $R_b$, $R_c$ and $R_d$ represent hydrogen atoms; -N represents a pyrrolidine ring substituted by =O, =CHCN or $=CHCO_2CH_3$; X represents $-CH_2-$; and Ar represents halosubstituted phenyl; and physiologically acceptable salts or solvates thereof.

8. A method according to Claim 1 wherein $R_a$ and $R_c$ represent hydrogen atoms and $R_b$ and $R_d$ together form -CH=CH-CH=CH-; -N represents a pyrrolidine ring substituted by -OH; X represents $-CH_2-$; and Ar represents halosubtituted phenyl; and physiologically acceptable salts or solvates thereof.

European Patent Office

# EUROPEAN SEARCH REPORT

Application Number

EP 89 30 1771

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 232 612 (DR. L. ZAMBELETTI S.P.A.)<br>* claims 1,3,6,7,10-14 *<br>--- | 1,2,4-6,9-11 | C 07 D 401/06<br>C 07 D 413/06<br>C 07 D 211/26<br>C 07 D 217/14<br>A 61 K 31/445<br>A 61 K 31/47 |
| A | EP-A-0 232 989 (DR. L. ZAMBELETTI S.P.A.)<br>* claims 1,3,7,9-12 *<br>--- | 1,3-6,9-11 | |
| A | EP-A-0 052 311 (STERLING DRUG INC.)<br>* formula VI; examples 1B,1C,1D *<br>----- | 1,2 | |
| | | | **TECHNICAL FIELDS SEARCHED (Int. Cl.4)** |
| | | | C 07 D 211/00<br>C 07 D 217/00<br>C 07 D 401/00<br>C 07 D 413/00 |

The present search report has been drawn up for all claims

| Place of search | Date of completion of the search | Examiner |
|---|---|---|
| BERLIN | 22-05-1989 | HASS C V F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)